(19) Europäisches Patentamt
European Patent Office
Office européen des brevets

(11) Veröffentlichungsnummer : **0 069 907**
**B1**

(12) # EUROPÄISCHE PATENTSCHRIFT

(45) Veröffentlichungstag der Patentschrift : **28.08.85**

(51) Int. Cl.⁴ : **C 07 C 85/24**, C 07 C 87/60

(21) Anmeldenummer : **82105708.0**

(22) Anmeldetag : **28.06.82**

(54) **Verfahren zur Herstellung von m-chlorsubstituierten Anilinen.**

(30) Priorität : **09.07.81 DE 3127026**

(43) Veröffentlichungstag der Anmeldung : **19.01.83 Patentblatt 83/03**

(45) Bekanntmachung des Hinweises auf die Patenterteilung : **28.08.85 Patentblatt 85/35**

(84) Benannte Vertragsstaaten : **BE CH DE FR GB LI**

(56) Entgegenhaltungen :
**EP-A- 0 015 219**
**EP-A- 0 051 782**

(73) Patentinhaber : **BAYER AG**
**Konzernverwaltung RP Patentabteilung**
**D-5090 Leverkusen 1 Bayerwerk (DE)**

(72) Erfinder : **Wedemeyer, Karlfried, Dr.**
**Bilharzstrasse 7**
**D-5000 Köln 80 (DE)**
Erfinder : **Hagedorn, Ferdinand, Dr.**
**Domblick 16**
**D-5090 Leverkusen 31 (DE)**
Erfinder : **Evertz, Werner**
**Wolffstrasse 1**
**D-4019 Monheim (DE).**

**EP 0 069 907 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von m-chlorsubstituierten Anilinen durch selektive Enthalogenierung von höherchlorierten Anilinen.

Aus der DE-PS-2 503 187 und der DE-PS 2 503 187 ist es bekannt, m-chlorsubstituierte Aniline durch selektive Enthalogenierung von höherhalogenierten Anilinen in Lösung in saurem Medium in Gegenwart von Edelmetallkatalysatoren und gegebenenfalls in Gegenwart von Schwefel und/oder Schwefelverbindungen herzustellen.

Die in der DE-PS-2 503 145 und der DE-PS-2 503 187 beschriebenen Verfahren arbeiten allerdings nur bei hohen Temperaturen und Drücken, d. h. bei Temperaturen von über 200 °C und Drücken von über 60 bar wirtschaftlich (vgl. hierzu auch die Beispiele der genannten Patentschriften). Da die dabei angewandten hohen Reaktionstemperaturen und Drücke z. B. die Behältermaterialien besonders stark beanspruchen, wird die technische Durchführung dieser Verfahren erschwert.

Ferner ist bekannt (deutsche Patentanmeldung P 3 042 242.5) m-halogensubstituierte Aniline durch mit Edelmetallkontakten katalysierte hydrierrende Enthalogenierung von halogensubstituierten Anilinen in Gegenwart von Jodwasserstoff und bei gleichzeitiger Gegenwart von gegegenenfalls substituierten Phenolen herzustellen.

Weiterhin ist aus der DE-OS-3 003 960 ein Verfahren zur Herstellung von m-chlorsubstituierten Anilinen bekannt, bei dem die hydrierende Enthalogenierung in Gegenwart einer stark erhöhten Chloridionen-Konzentration durchgeführt wird. Dieses Verfahren hat den Nachtein, daß nur geringe Konzentrationen der zur Enthalogenierung eingesetzten Verbindung in Lösungen mit relativ hoher Chloridionen-Konzentration angewendet werden können, was zu schlechten Raum/Zeit-Ausbeuten und zu erhöhter Korrosion der Apparaturen führt. Das einzige Beispiel dieser Anmeldung läßt vermuten, daß nur in Gegenwart von Lithiumchlorid gute Ergebnisse erzielt werden. Bei dieser Verfahrensweise erhält man jedoch große Mengen an nicht-trennbaren Salzgemischen, deren Aufarbeitung und Beseitigung besondere ökologische Probleme aufwirft.

In der europäischen Patentanmeldung 0 015 219 wird ein Verfahren beansprucht, bei dem die katalytisch hydrierende Enthalogenierung in Gegenwart von Jodid- oder Bromidionen durchgeführt wird. Dieses Verfahren hat den Nachteil, daß man zusätzlich zum während der Reaktion entstehenden Chlorwasserstoff noch größere Mengen an Salzsäure zusetzen muß, wodurch einmal ein erhöhter Zwangsanfall an Chlorwasserstoff bzw. daraus durch Neutralisation gebildetem Salz entsteht, zum anderen die Korrosionsgefahr für die verwendeten Apparaturen erhöht wird. Ein weiterer wesentlicher Nachteil besteht darin, daß nur geringe Konzentrationen der zur Enthalogenierung eingesetzten Verbindung im Reaktionsgemisch angewendet werden können (vgl. Beispiele 1 bis 15 : max. 3 Gew.-%). Will man jedoch größere Mengen an Ausgangsprodukt enthalogenieren, so ist es erforderlich, eine große Menge an Katalysator, bezogen auf die zu enthalogenierende Verbindung, einzeusetzen (vgl. Beispiel 16 ; Konzentration an Tetrachlornitrobenzol im Gemisch : 12 %, Katalysatormenge : 57 Gew.-%, bezogen auf Tetrachlornitrobenzol). Die Verwendung von Jodid- bzw. Bromid-Verbindungen bei diesem Verfahren macht aus ökologischen und wirtschaftlichen Gründen eine Abtrennung dieser Verbindungen aus dem Reaktionsgemisch sowie eine Aufarbeitung zur Wiedergewinnung erforderlich, was die Durchführbarkeit des Verfahrens erschwert.

Wie den obigen Ausführungen zu entnehmen ist, handelt es sich bei dem in der europäischen Patentanmeldung 0 015 219 beschriebenen Verfahren um ein technisch und wirtschaftlich wenig geeignetes Verfahren.

Es wurde nun ein Verfahren zur Herstellung von m-chlorsubstituierten Anilinen durch Umsetzung von Anilinen der Formel

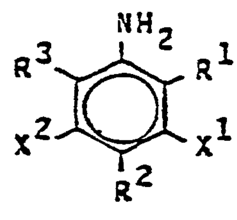

(I)

worin

$X^1$ und $X^2$ gleich oder verschieden sind und für Chlor, Wasserstoff oder einen gegebenenfalls substituierten Alkyl-, Aryl-, Aralkyl-, Alkoxy- oder Aralkoxyrest stehen, wobei bei der Herstellung von 3-Chloranilinen einer der Reste $X^1$ oder $X^2$ für Chlor steht und bei der Herstellung von 3,5-Dichloranilinen $X^1$ und $X^2$ für Chlor stehen,

$R^1$, $R^2$, $R^3$ gleich oder verschieden sind und für Chlor, Wasserstoff oder einen gegebenenfalls substituierten Alkyl-, Aryl-, Aralkyl-, Alkoxy- oder Aryloxyrest stehen, wobei wenigstens einer der Reste $R^1$, $R^2$ oder $R^3$ für Chlor steht,

mit Wasserstoff im sauren Medium in Gegenwart von gegebenenfalls auf Trägern aufgebrachten

Edelmetallen in elementarer oder gebunder Form und gegebenenfalls in Gegenwart von inerten, organischen Lösungs- und/oder Verdünnungsmitteln und/oder Wasser bei erhöhter Temperatur und erhöhten Drücken gefunden, das dadurch gekennzeichnet ist, daß man die Umsetzung in Gegenwart von gegebenenfalls substituierten Phenolen und in Abwesenheit von Iodwasserstoff und/oder Iodwasserstoff liefernden Verbindungen durchführt.

Nach dem erfindungsgemäßen Verfahren erhält man m-chlorsubstituierte Aniline der Formel (II)

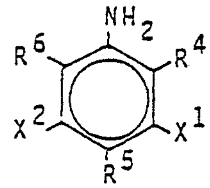

(II)

worin

$X^1$ und $X^2$ die oben angegebene Bedeutung haben, und

$R^4$, $R^5$ und $R^6$ gleich oder verschieden sind und für Wasserstoff oder für einen gegebenenfalls substituierten Alkyl-, Aryl-, Aralkyl-, Alkoxy- oder Aryloxyrest stehen.

Gegenbenenfalls substituierte aliphatische Reste ($R^1$, $R^2$, $R^3$, $R^4$, $R^5$ und $R^6$) können beispielsweise geradkettige oder verzweigte aliphatische Reste mit 1 bis 12, bevorzugt mit 1 bis zu 6 Kohlenstoffatomen und cycloaliphatische Reste mit 5 bis 8, bevorzugt 5 und 6, Kohlenstoffatomen im Ring sein. Beispielsweise seien der Methyl-, der Ethyl-, der Propyl-, der Isopropyl-, der Butyl-, der Pentyl-, der Hexyl-, der Octyl-, der Nonyl-, der Decyl-, der Dodecyl-, der Cyclopentyl-, der Cyclohexyl-, der Cycloheptyl- und der Cyclooctylrest genannt.

Gegebenenfalls substituierte aromatische Reste ($R^1$, $R^2$, $R^3$, $R^4$, $R^5$ und $R^6$) können Reste aus der Benzolreihe, bevorzugt der Phenyl- oder der Naphthylrest, sein.

Gegebenenfalls substituierte Aralkylreste ($R^1$, $R^2$, $R^3$, $R^4$, $R^5$ und $R^6$) können beispielsweise solche mit 7 bis 18 Kohlenstoffatomen sein, deren aliphatischer Teil 1 bis 6 Kohlenstoffatome, vorzugsweise 1 bis 3 Kohlenstoffatome, enthält und deren aromatischer Teil einen Rest der Benzolreihe, vorzugsweise den Phenyl- oder den Naphthylrest, darstellt. Beispielsweise seien die folgenden Aralkylreste genannt : der Benzyl-, der β-Phenyl-ethyl, der γ-Phenyl-propyl, der β-Phenyl-n-hexyl-, der β-[Naphthyl-(1)]-ethyl-, der ω-Butyl-phenyl-, der ω-Pentyl-phenyl- und der ω-Hexyl-phenyl-rest.

Für gegebenenfalls substituierte Alkoxyreste ($R^1$, $R^2$, $R^3$, $R^4$, $R^5$ und $R^6$) können sowohl geradkettige und verzweigte Reste mit 1 bis 12, bevorzugt 1 bis 6, Kohlenstoffatomen, als auch cycloaliphatische Reste mit 5 und 6 Kohlenstoffatomen im Ring stehen. Beispielsweise sei der Methoxy-, der Ethoxy-, der Propoxy-, der Isopropoxy-, der Butoxy-, der tert.-Butoxy-, der Pentoxy-, der Hexoxy-, der Octoxy-, der Nonoxy-, der Decoxy-, der Dodecoxy-, der Cyclopentoxy- und der Cyclohexoxy-rest genannt.

Als gegebenenfalls substituierte Aryloxyreste ($R^1$, $R^2$, $R^3$, $R^4$, $R^5$ und $R^6$) seien Reste aus der Benzolreihe, bevorzugt der Phenoxyrest, genannt.

Als Substituenten der vorstehend aufgeführten Alkyl-, Aryl-, Aralkyl-, Alkoxy- oder Aralkoxyreste kommen beispielsweise die Aminogruppe, die Hydroxygruppe, geradkettige oder verzweigte Alkylreste mit bis zu 12, bevorzugt mit bis zu 6 Kohlenstoffstomen, cycloaliphatische Reste, bevorzugt mit 5 und 6 Kohlenstoffatomen im Ring, und Arylreste, bevorzugt der Phenylrest, in Frage.

Besonders bevorzugte Chloraniline, die für das erfindungsgemäße Verfahren eingesetzt werden können, sind Verbindungen der Formel (III)

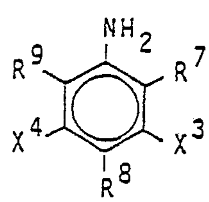

(III)

worin

$X^3$ und $X^4$ gleich oder verschieden sind und für Chlor oder Wasserstoff stehen, wobei bei der Herstellung von 3-Chloranilinen einer der Reste $X^3$ oder $X^4$ für Chlor steht und bei der Herstellung von 3,5-Dichloranilinen $X^3$ und $X^4$ für Chlor stehen,

$R^7$, $R^8$ und $R^9$ gleich oder verschieden sind und für Chlor, Wasserstoff, die Methyl- oder die Phenylgruppe oder den Rest

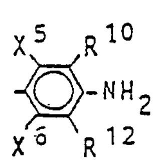

oder

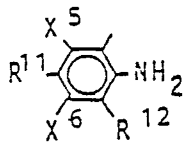

stehen, worin

$X^5$ und $X^6$ gleich oder verschieden sind und für Chlor oder Wasserstoff stehen,

$R^{10}$, $R^{11}$ und $R^{12}$ gleich oder verschieden sind und für Chlor, Wasserstoff, die Methyl- oder die Phenylgruppe stehen, wobei wenigstens einer der Reste $R^7$, $R^8$ oder $R^9$ für Chlor steht.

Die Polychloraniline der Formel (I), die für das erfindungsgemäße Verfahren Verwendung finden können, sind bekannt und leicht zugänglich.

Beispielsweise seien genannt :

2,3-Dichlor-anilin, 2,5-Dichlor-anilin,
3,4-Dichlor-anilin, 2,3,4-Trichlor-anilin,
2,3,5-Trichlor-anilin, 2,4,5-Trichlor-anilin,
2,3,6-Trichlor-anilin, 3,4,5-Trichlor-anilin,
2,3,4,6-Tetrachlor-anilin, 2,3,4,5-Tetrachlor-anilin,
2,3,5,6-Tetrachlor-anilin, Pentachlor-anilin,
4,5,6-Trichlor-2-methyl-anilin,
2,5-Dichlor-4-methyl-anilin,
2,3,5,6-Tetrachlor-4-methyl-anilin,
2,5-Dichlor-3,4-dimethyl-anilin,
2,5-Dichlor-4-äthyl-anilin,
2,5-Dichlor-4-propyl-anilin,
3,4,6-Trichlor-2-benzyl-anilin,
2,2'-Diamino-3,5,6,3',5',6'-hexachlor-diphenylmethan,
3,4,5-Trichlor-2-amino-diphenyl,
4,4'-Diamino-octachlor-diphenyl,
3,4-Dichlor-2-methoxy-anilin,
3,6-Dichlor-2-methoxy-anilin,
4,5-Dichlor-2-methoxy-anilin,
5,6-Dichlor-2-methoxy-anilin,
3,4,6-Trichlor-2-methoxy-anilin,
3,4,5-Trichlor-2-methoxy-anilin,
3,4,5,6-Tetrachlor-2-methoxy-anilin,
4,5-Dichlor-3-methoxy-anilin,
5,6-Dichlor-3-methoxy-anilin,
2,5-Dichlor-3-methoxy-anilin,
4,5,6-Trichlor-3-methoxy-anilin,
2,4,5,6-Tetrachlor-3-methoxy-anilin,
2,3-Dichlor-4-methoxy-anilin,
2,5-Dichlor-4-methoxy-anilin,
2,3,6-Trichlor-4-methoxy-anilin,
2,3,5-Trichlor-4-methoxy-anilin,
2,3,5,6-Tetrachlor-4-methoxy-anilin,
4,5-Dichlor-2-phenoxy-anilin,
3,4,5,6-Tetrachlor-2-phenoxy-anilin,
2,4,5,6-Tetrachlor-3-phenoxy-anilin,
2,5-Dichlor-4-phenoxy-anilin,
2,3,5,6-Tetrachlor-4-phenoxy-anilin.

Das erfindungsgemäße Verfahren wird in Gegenwart von gegebenenfalls substituierten Phenolen als Reaktionsmedium durchgeführt.

Als gegebenenfalls substituierte Phenole kommen solche in Frage, die ein- oder mehrfach durch Alkylgruppen mit bis zu 4, bevorzugt 1 bis 3, Kohlenstoffatomen und/oder durch Halogen, bevorzugt durch Chlor, substituiert sind.

Zum Beispiel werden genannt : Phenol, o-, m-, p-Kresol, 2,3-, 2,4-, 2,5-, 2,6- und 3,5-Xylenol, bevorzugt Phenol, o-, m- und p-Kresol.

Die gegebenenfalls substituierten Phenole können sowohl einzeln als auch im Gemisch untereinander in das erfindungsgemäße Verfahren eingesetzt werden, z. B. m-/p-Kresol-Gemische.

Die Menge der in das erfindungsgemäße Verfahren einzusetzenden, gegebenenfalls substituierten Phenole kann in weiten Bereichen variiert werden. Üblicherweise werden 1 bis 90 Gew.-%, bevorzugt 30 bis 70 Gew.-%, gegebenenfalls substituierte Phenole, bezogen auf das Gesamtgewicht des Reaktionsgemisches, in das erfindungsgemäße Verfahren eingesetzt.

Die gegebenenfalls substituierten Phenole werden vorzugsweise unverdünnt in das erfindungsgemäße Verfahren eingesetzt. Es ist jedoch auch möglich, die Phenole in Lösung oder Verdünnung in einem unter den Reaktionsbedingungen inerten organischen Lösungs- und/oder Verdünnungsmittel oder in Wasser einzusetzen.

Als Edelmetalle seien die Elemente der 8. Gruppe des Periodensystems der Elemente (Mendelejew), wie Ruthenium, Rhodium, Palladium, Osmium, Iridium und Platin, bevorzugt Palladium und Platin, genannt.

Als Edelmetalle in gebundener Form können beispielsweise die Oxide, Sulfide und/oder Polysulfide eingesetzt werden.

Die Katalysatoren nach dem erfindungsgemäßen Verfahren können auch auf Trägermaterialien angewendet werden. Dafür kommen alle an sich bekannten Trägermaterialien in Frage, soweit sie gegen Wasser und Säuren inert sind. Als solche seien Bariumsulfat und Aktivkohle, bevorzugt Aktivkohle, genannt.

Die Herstellung des Edelmetallkatalysators auf einem Trägermaterial kann in an sich bekannter Weise erfolgen. Beispielsweise wird das Trägermaterial in der wäßrigen Lösung des Edelmetalls aufgeschlämmt und dann das Edelmetall durch Zugabe eines Reduktionsmittels, wie Wasserstoff oder Hydrazin, auf das Trägermaterial gefällt.

Besonders bei der kontinuierlichen Durchführung des erfindungsgemäßen Verfahrens ist es vorteilhaft, den Edelmetallkatalysator auf einem Trägermaterial im Reaktionsraum als Festbett- oder Fließbett-Katalysator anzuordnen.

Die Katalysatoren, die zur Durchführung des erfindungsgemäßen Verfahrens eingesetzt werden, erhalten auch bei mehrmalig wiederholter Verwendung oder bei kontinuierlicher Durchführung des erfindungsgemäßen Verfahrens über lange Zeit ihre Aktivität und ihre Selektivität und ergeben g leichbleibend hohe Ausbeuten.

Die Menge des Katalysators, der zur Durchführung des erfindungsgemäßen Verfahrens eingesetzt wird, beträgt im allgemeinen 0,1 bis 2 Gew.-%, bevorzugt 1 bis 1,5 Gew.-%, bezogen auf das als Ausgangsmaterial verwendete Anilin. Bei Verwendung eines auf einen Träger aufgebrachten Katalysators werden üblicherweise 1 bis 20 Gew.-%, bevorzugt 10 bis 20 Gew.-%, bezogen auf das Ausgangsmaterial, eingesetzt.

Das erfindungsgemäße Verfahren wird gegebenenfalls in Gegenwart von weiteren inerten organischen Lösungs- und/oder Verdünnungsmitteln und/oder in Gegenwart von Wasser durchgeführt. Als inerte, organische Lösungs- und/oder Verdünnungsmittel werden beispielsweise Benzol, Chlorbenzol, o-Dichlorbenzol, Toluol und Xylol, vorzugsweise Toluol, genannt.

Im allgemeinen wird das erfindungsgemäße Verfahren in saurem Medium durchgeführt. Das saure Medium kann durch den bei der Reaktion entstehenden Halogenwasserstoff erzeugt werden.

Bei dem Arbeiten in Gegenwart von Wasser wird das erfindungsgemäße Verfahren im allgemeinen bei einem pH-Wert von kleiner als 4, bevorzugt kleiner als 1, durchgeführt.

Zur einstellung des sauren Mediums auf einen pH-Wert von < 1 kann man dem Gemisch vor Beginn der Reaktion eine kleine Menge Chlorwasserstoff zusetzen. Die Menge des zugesetzten Chlorwasserstoffs wird vorzugsweise sehr gering gehalten, um einen erhöhten Zwangsanfall an Chlorwasserstoff bzw. dessen Salzen bei der Aufarbeitung zu vermeiden.

In einer besonderen Ausführungsform des Verfahrens wird die Einstellung des sauren Mediums durch Wiederverwenden eines gegebenenfalls auf einem Träger aufgebrachten Edelmetallkatalysators aus einem vorausgegangen Reaktionsansatz erreicht, wobei der Katalysator vor dem Wiedereinsatz aus dem stark sauren Reaktionsgemisch eines vorangegangenen Ansatzes abgetrennt und gegebenenfalls mit heißem Wasser oder heißer, verdünnter Salzsäure gewaschen wird.

Das erfindungsgemäße Verfahren kann anhand der folgenden Reaktionsgleichung für die Entchlorierung von Pentachloranilin zu 3,5-Dichloranilin erläutert werden :

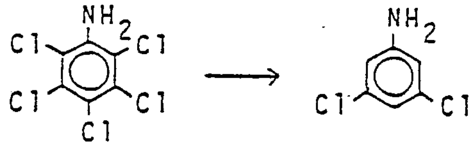

Nach dem erfindungsgemäßen Verfahren ist es möglich, nicht nur von z. B. reinen Tetrachloranilinen oder Pentachloranilinen auszugehen, sondern auch von Gemischen aus Tetrachloranilinen und Pentachloranilin, wie sie bei der technischen Herstellung aus Tetrachlorbenzol durch Nitrieren und katalytische Reduktion der Nitrogruppe anfallen.

Weiterhin ist es möglich, als Ausgangsmaterial anstelle der chlorierten Aniline die entsprechenden

Nitrochlorverbindungen einzusetzen und in einer Eintopfreaktion die Reduktion der Nitrogruppe und die hydrierende Enthalogenierung durchzuführen.

Im allgemeinen wird das erfindungsgemäße Verfahren so durchgeführt, daß man das Ausgangsmaterial, das Phenol, gegebenenfalls ein Lösungs- oder Verdünnungsmittel und den Katalysator in einem säurefesten Autoklav, z. B. einem Autoklav aus Emaille oder Tantal, vorlegt und nach Verschließen des Autoklavs die Luft mit Stickstoff und anschließend den Stickstoff mit Wasserstoff verdrängt.

Zur Durchführung der Reaktion wird der Wasserstoff gasförmig in das Reaktionsgemisch geleitet. Im allgemeinen arbeitet man bei einem Gesamtdruck von 5 bis 100 bar, bevorzugt bei 10 bis 60 bar, besonders bevorzugt bei 15 bis 50 bar.

Das erfindungsgemäße Verfahren wird im allgemeinen bei einer Temperatur von 100 bis 250 °C, bevorzugt bei 120 bis 200 °C, besonders bevorzugt bei 140 bis 190 °C, durchgeführt.

Die Reaktionsdauer hängt u. a. von der angewandten Reaktionstemperatur und dem angewandten Wasserstoffdruck ab und liegt bei 180 °C bei etwa 2 Stunden.

Nach beendeter Reaktion kann das Gemisch in an sich bekannter Weise aufgearbeitet werden. Zum Beispiel kann nach Verdünnen mit Wasser der Katalysator durch Absaugen des heißen Gemisches abgetrennt werden. Die m-chlorsubstituierten Aniline können anschließend durch Zugabe von z. B. Alkalihydroxidlösung freigesetzt und mit einem mit Wasser nicht-mischbaren Lösungsmittel, z. B. Methylenchlorid oder Toluol, extrahiert werden. Dabei bleibt das Phenol als Alkaliphenolat in Lösung. Aus dem Lösungsmittel können die Halogenaniline beispielsweise durch Destillation erhalten werden.

Bei dem Einsatz eines nicht mit Wasser mischbaren Lösungs- und/oder Verdünnungsmittels können die 3-Chlor- bzw. die 3,5-Dichloraniline durch Zugabe von wäßrigem Alkalihydroxid freigesetzt werden. Anschließend kann das organische Lösungsmittel abgetrennt und daraus das m-chlorsubstituierte Anilin, z. B. durch Destillation, isoliert werden.

Eine besondere Form der Aufarbeitung kann darin bestehen, daß man z. B. nach Verdünnen des Reaktionsgemisches mit Wasser, Absaugen des Katalysators und Neutralisation mit z. B. Natronlauge die organische Phase, gegebenenfalls mit Hilfe eines organischen Lösungsmittels wie Toluol, abtrennt und durch Destillation das jeweilige chlorsubstituierte Anilin gewinnt.

Das erfindungsgemäß Verfahren kann sowohl diskontinuierlich als auch kontinuierlich durchgeführt werden.

Das erfindungsgemäße Verfahren hat den Vorteil, daß ohne zusätzliche Zugabe von Salzen, wie Alkali- oder Erdalkalichloriden, sowie ohne Verwendung von Jod- oder Bromionen, deren Wiedergewinnung zusätzlichen Aufwand erfordert (s. europäische Patentanmeldung 0 015 219), ferner, daß ohne die Verwendung von Schwermetallsalzen, wie sie z. B. in der europäischen Patentanmeldung 0 015 220 genannt werden, unter milden Reaktionsbedingungen bei hohen Konzentrationen an Einsatzmaterial die selektive Entchlorierung mit hoher Ausbeute durchgeführt werden kann.

Ein weiterer Vorteil des erfindungsgemäßen Verfahrens ist, daß als Ausgangsmaterial auch schwer trennbare Chloranilin-Gemische verwendet werden können, die neben den Polychloranilinen, die in m-Stellung zur Aminogruppe durch Chlor substituiert sind, weitere Chlor- oder Polychloraniline enthalten, bei denen kein Chlor in m-Stellung zur Aminogruppe steht. Diese Verbindungen werden nach dem erfindungsgemäßen Verfahren zu Anilin entchloriert, das sich leicht destillativ abtrennen läßt. Dagegen ist die Auftrennung eines Polychloranilin-Gemisches umständlich und langwierig.

Ferner kann nach dem erfindungsgemäßen Verfahren ein Gemisch aus Tetrachloranilinen und Pentachloranilin, wie es aus einem technisch hergestellten Tetrachlorbenzol-Gemisch durch Nitrieren und katalytische Reduktion der Nitrogruppe zugänglich ist, selektiv und mit hoher Ausbeute zu 3,5-Dichloranilin enthalogeniert werden.

Es muß weiterhin als vorteilhaft angesehen werden, daß das bei der Enthalogenierungsreaktion eingesetzte Phenol unter den genannten Reaktionsbedingungen und in Gegenwart von Edelmetallkatalysatoren mit Wasserstoff nicht zu Cyclohexanol oder Cyclohexanon durch Kernhydrierung umgesetzt wird. Dabei kann das Phenol jeweils durch eine einfache Trennoperation, wie Extraktion oder Destillation, wiedergewonnen und wiederverwendet werden. Der Einsatz von gegebenenfalls substituierten Phenolen bei dem erfindungsgemäßen Verfahren bietet noch einen weiteren wesentlichen Vorteil, da die zu enthalogenierende Verbindung in sehr hoher Konzentration bei gleichzeitig geringer Konzentration an Edelmetallkatalysator eingesetzt werden kann. Dadurch werden besonders hohe, bislang nicht erreichte Raum/Zeit-Ausbeuten erzielt.

Als durch Chlor m-substituierte Aniline der Formel (II), die nach dem erfindungsgemäßen Verfahren hergestellt werden können, seien beispielsweise genannt :

3-Chlor-anilin,
3,5-Dichlor-anilin,
5-Chlor-2-methyl-anilin,
5-Chlor-3-methyl-anilin,
3-Chlor-4-methyl-anilin,
3,5-Dichlor-4-methyl-anilin,
5-Chlor-3,4-dimethyl-anilin,
3-Chlor-4-ethyl-anilin,

3-Chlor-2-benzyl-anilin,
4,4'-Diamino-2,6,2',6'-tetrachlor-diphenyl,
3-Chlor-2-methoxy-anilin,
5-Chlor-2-methoxy-anilin,
3,5-Dichlor-2-methoxy-anilin,
3-Chlor-4-methoxy-anilin,
5-Chlor-3-methoxy-anilin,
3,5-Dichlor-4-methoxy-anilin,
3-Chlor-2-phenoxy-anilin,
3,5-Dichlor-2-phenoxy-anilin,
3,5-Dichlor-4-phenoxy-anilin.

Die nach dem erfindungsgemäßen Verfahren erhältlichen 3-Chlor- bzw. 3,5-Dichloraniline sind bekannte Zwischenprodukte und können zur Herstellung von Pflanzenschutzmitteln verwendet werden (DE-PS-1 034 912, DE-OS-2 021 327, DE-OS-1 812 206, DE-OS-1 958 183, US-PS-2 906 614, US-PS 2 655 445, 1 034 912, DE-OS-2 021 327, DE-OS-1 812 206, DE-OS-1 958 183, US-PS-2 906 614, US-PS 2 655 445,
Die nachfolgenden Beispiele sollen das erfindungsgemäße Verfahren verdeutlichen.

## Beispiel 1

In einem Tantal-Autoklaven werden 131 Teile eines Gemisches von 2,3,4,5- und 2,3,5,6-Tetrachloranilin sowie Pentachloranilin im Gewichtsprozent-Verhältnis 38 : 60 : 2 und 200 Teile Phenol an 20 Teilen Palladium-Kohle-Kontakt (1 %ig) mit Wasserstoff bei einem Druck von max. 50 bar und bei 170 °C innerhalb 10 Stunden unter Rühren umgesetzt. Nach dem Abkühlen und Entspannen wird das Reaktionsgemisch mit 500 Teilen Wasser verdünnt, vom Kontakt abgetrennt und nach Zugabe von Toluol mit Natronlauge alkalisch gestellt. Die durch Schichttrennen gewonnene Lösung von 3,5-Dichloranilin in Toluol wird destilliert. Ausbeute : 90 g (= 98 %) eines 99 %igen 3,5-Dichloranilins. Aus der wäßrigen Phase läßt sich durch Ansäuern und Ausschütteln mit Toluol das Phenol wiedergewinnen. Der Katalysator konnte 20 mal wieder verwendet werden, ohne daß er seine Aktivität einbüßte.

## Beispiel 2

In einem Tantal-Autoklaven (0,85 l) werden 200 Teile Phenol, 197 Teile eines Gemisches von 2,3,4,5- und 2,3,5,6-Tetrachloranilin sowie Pentachloranilin im Gewichtsprozent-Verhältnis von etwa 38 : 60 : 2 an 20 Teilen Palladium-Kohle-Kontakt (1 %ig) mit Wasserstoff bei einem Druck von max. 50 bar und bei 175 °C innerhalb 10 Stunden unter Rühren umgesetzt. Nach dem Abkühlen und Entspannen wird das Reaktionsgemisch mit 800 Teilen Wasser verdünnt, der Katalysator wird von der heißen Lösung abgesaugt und nach Waschen mit etwas heißem Wasser in einem weiteren Reaktionsansatz wiederverwendet. Die vom Kontakt befreite Lösung aus 3,5-Dichloranilin-hydrochlorid, Phenol und Wasser wird mit Natronlauge neutralisiert. Mit Hilfe von Toluol trennt man 3,5-Dichloranilin und Phenol von der Wasserphase ab und destilliert die organische Lösung. Man erhält ein 98 %iges 3,5-Dichloranilin in einer Ausbeute von 95 % bei vollständigen Tetrachlor-/Pentachloranilin-Umsatz.

## Beispiel 3

40 Teile 2,3,4,5-Tetrachloranilin, 150 Teile Phenol und 12 g eines bereits für diese Reaktion benutzten feuchten Palladium-Kohle-Katalysators (1 %ig, 8 g trocken) werden vermischt und in einem Tantal-Autoklaven nach Aufpressen von Wasserstoff bis zu einem Gesamtdruck von 16 bis max. 18 bar bei 165 °C 6 Stunden gerührt. Nach Abkühlen und Entspannen des Druckbehälters verdünnt man das Reaktionsgemisch mit Wasser, gibt Toluol zu und stellt das Gemisch mit Natronlauge alkalisch. Durch Absaugen trennt man den Katalysator ab. Die Toluolphase destilliert man und erhält 27,7 Teile (= 99 % Ausbeute) 98,5 %iges 3,5-Dichloranilin.

## Beispiel 4

Ein Gemisch aus 200 Teilen Phenol, 150 Teilen eines Tetrachlornitrobenzol/Pentachlornitrobenzol-Gemisches und 20 g Palladium-Kohle-Katalysator, 1 %ig, wird in einem Tantal-Autoklav zunächst bei 60 bis 70 °C mit Wasserstoff bei 10 bar unter Rühren innerhalb 6 Stunden reduziert. Danach erhöht man die Temperatur auf 170 °C und führt unter weiterem Rühren bei einem Gesamtdruck von max. 50 bar die Enthalogenierung innerhalb 8 Stunden durch. Nach Aufarbeitung des Reaktionsgemisches erhält man durch Destillation 86 Teile 3,5-Dichloranilin, 94 % Ausbeute.

## Beispiel 5

In einem Tantal-Autoklav werden 81 Teile 2,3-Dichloranilin, 99,1 %ig, und 180 Teile Phenol mit 25

Teilen eines salzsäure-feuchten Palladium-Kohle-Kontakts (1 %ig, 20 g trocken) vermischt und mit Wasserstoff bei einem Gesamtdruck von max. 50 bar unter Rühren bei 175° innerhalb 4 Stunden umgesetzt. Nach dem Abkühlen und Entspannen des Druckbehälters verdünnt man das Gemisch mit 400 Teilen Wasser und 200 Teilen konz. wäßriger Natronlauge und erwärmt das Gemisch nach Zugabe von 200 Teilen Toluol auf 90 °C. Der Katalysator wird abgesaugt und mit heißem Toluol ausgespült. Die vereinigten Toluollösungen werden destilliert, wobei man 60,5 Teile 3-Chloranilin (= 96 % d. Th.) erhält.

Beispiel 6

61,5 Teile 2,3,5,6-Tetrachlor-4-methyl-anilin, 180 Teile Phenol und 15 Teile eines 1 %igen Palladium-Kohle-Katalysators werden in einem Tantal-Autoklav in Gegenwart einer kleinen Menge Salzsäure unter Rühren bei 180° und mit Wasserstoff bei max. 50 bar innerhalb 5 Stunden umgesetzt. Nach dem Abkühlen und Entspannen des Druckbehälters verdünnt man das Gemisch mit 300 Teilen Wasser und 200 Teilen Toluol, stellt es alkalisch, erhitzt es auf 90° und trennt vom Kontakt ab. Der Katalysator wird mit Toluol ausgekocht, die vereinigten Toluol-Lösungen werden destilliert. Man erhält 44 Teile 3,5-Dichlor-4-methylanilin, Kp 146-155°/18 mbar, 98 %ig ; Ausbeute : 98 % d. Th.

Beispiel 7

In einem Tantal-Autoklav werden 131 Teile eines Tetrachlor-/Pentachloranilin-Isomerengemisches (gemäß Beispiel 2), 200 Teile o-Kresol und 20 Teile eines Pd-Kohle-Katalysators (1 %ig) sowie 5 Teile konz. Salzsäure unter Rühren bei 175° und 50 bar mit Wasserstoff innerhalb 10 Stunden umgesetzt. Nach Abkühlen und Entspannen des Autoklaven wird das Gemisch mit 200 Teilen Toluol, 400 Teilen Wasser und 250 Teilen wäßriger konz. Natronlauge alkalisch gestellt und bis zum Sieden erhitzt, danach trennt man den Kontakt durch Absaugen ab. Die Toluollösungen des 3,5-Dichloranilins werden nach Ausschütteln der wäßrigen, alkalischen Phase mit Toluol destilliert. Man erhält 85 Teile eines 98 %igen 3,5-Dichloranilins, Kp 130-136°/18 mbar.

**Patentansprüche**

1. Verfahren zur Herstellung von m-chlorsubstituierten Anilinen durch Umsetzung von Anilinen der Formel

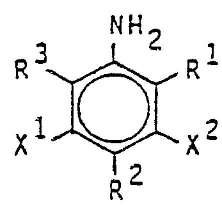

worin

$X^1$ und $X^2$ gleich oder verschieden sind und für Chlor, Wasserstoff oder einen gegebenenfalls substituierten Alkyl-, Aryl-, Aralkyl-, Alkoxy- oder Aralkoxyrest stehen, wobei bei der Herstellung von 3-Chloranilinen einer der Reste $X^1$ oder $X^2$ für Chlor steht und bei der Herstellung von 3,5-Dichloranilinen $X^1$ und $X^2$ für Chlor stehen,

$R^1$, $R^2$, $R^3$ gleich oder verschieden sind und für Chlor, Wasserstoff oder einen gegebenenfalls substituierten Alkyl-, Aryl-, Aralkyl-, Alkoxy- oder Aryloxyrest stehen, wobei wenigstens einer der Reste $R^1$, $R^2$ oder $R^3$ für Chlor steht,

mit Wasserstoff im sauren Medium in Gegenwart von gegebenenfalls auf Trägern aufgebrachten Edelmetallen in elementarer oder gebundener Form und gegebenenfalls in Gegenwart von inerten, organischen Lösungs- und/oder Verdünnungsmitteln und/oder Wasser bei erhöhten Drücken und erhöhter Temperatur, dadurch gekennzeichnet, daß man die Umsetzung in Gegenwart von gegebenenfalls substituierten Phenolen und in Abwesenheit von Iodwasserstoff und/oder Iodwasserstoff liefernden Verbindungen durchführt.

2. Verfahren nach Anspruch 1, dadurch gekennzeichnet, daß man als gegebenenfalls substituierte Phenole solche einsetzt, die ein- oder mehrfach durch Alkylgruppen mit bis zu 4 Kohlanstoffatomen und/oder durch Halogen substituiert sind.

3. Verfahren nach Ansprüchen 1 und 2, dadurch gekennzeichnet, daß man Phenol, o-, m-, und p-Kresol, 2,3-, 2,4-, 2,5-, 2,6- und/oder 3,5-Xylenol einsetzt.

4. Verfahren nach Ansprüchen 1 und 2, dadurch gekennzeichnet, daß man Phenol, o-, und m-, p-Kresol oder ein m-/p-Kresolgemisch einsetzt.

5. Verfahren nach Ansprüchen 1 bis 4, dadurch gekennzeichnet, daß man die gegebenenfalls

substituierten Phenole in Mengen von 1 bis 90 Gew.-%, bezogen auf das Gesamtgewicht des Reaktionsgemisches, einsetzt.

6. Verfahren nach Ansprüchen 1 bis 4, dadurch gekennzeichnet, daß man die gegebenenfalls substituierten Phenole in Mengen von 30 bis 70 Gew.-%, bezogen auf das Gesamtgewicht des Reaktionsgemisches, einsetzt.

7. Verfahren nach den Ansprüchen 1 bis 6, dadurch gekennzeichnet, daß der gegebenenfalls auf einem Träger aufgebrachte Edelmetallkatalysator nach beendeter Reaktion und Verdünnen des Reaktionsgemisches abgetrennt und entweder direkt oder nach einer Wäsche mit Wasser oder Salzsäure bei einem nachfolgenden Reaktionsansatz wieder eingesetzt wird.

**Claims**

1. Process for the preparation of anilines, which are m-substituted by chlorine, by the reaction of anilines of the formula

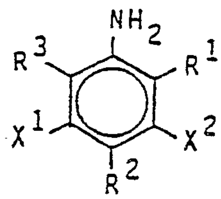

wherein

$X^1$ and $X^2$ are identical or different and represent chlorine, hydrogen or an optionally substituted alkyl, aryl, aralkyl, alkoxy or aralkoxy radical, one of the radicals $X^1$ or $X^2$ representing chlorine in the preparation of 3-chloroanilines, and $X^1$ and $X^2$ representing chlorine in the preparation of 3,5-dichloroanilines, and

$R^1$, $R^2$ and $R^3$ are identical or different and represent chlorine, hydrogen or an optionally substituted alkyl, aryl, aralkyl, alkoxy or aryloxy radical, at least one of the radicals $R^1$, $R^2$ or $R^3$ representing chlorine, with hydrogen in an acid medium in the presence of noble metals, in elementary or bonded form, which may be applied onto carriers, and if appropriate in the presence of inert, organic solvents and/or diluents and/or water, at elevated pressures and elevated temperature, characterised in that the reaction is carried out in the presence of optionally substituted phenols and in the absence of hydrogen iodide and/or compounds yielding hydrogen iodide.

2. Process according to Claim 1, characterised in that the optionally substituted phenols employed are those which are monosubstituted or polysubstituted by alkyl groups having up to 4 carbon atoms and/or by halogen.

3. Process according to Claims 1 and 2, characterised in that phenol, o-, m-, and p-cresol, and 2,3-, 2,4-, 2,5-, 2,6- and/or 3,5-xylenol are employed.

4. Process according to Claims 1 and 2, characterised in that phenol, o-, m-, p-cresol or an m-cresol/p-cresol mixture is employed.

5. Process according to Claims 1 to 4, characterised in that the optionally substituted phenols are employed in amounts of from 1 to 90 % by weight, relative to the total weight of the reaction mixture.

6. Process according to Claims 1 to 4, characterised in that the optionally substituted phenols are employed in amounts of from 30 to 70 % by weight, relative to the total weight of the reaction mixture.

7. Process according to Claims 1 to 6, characterised in that the noble metal catalyst which may be applied on a carrier is separated off after the reaction has ended and the reaction mixture has been diluted, and is re-used in a subsequent reaction mixture either directly or after washing with water or hydrochloric acid.

**Revendications**

1. Procédé de production d'anilines substituées en méta avec du chlore, par réaction d'anilines de formule

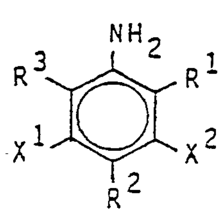

dans laquelle

$X^1$ et $X^2$ sont égaux ou différents et représentent du chlore, de l'hydrogène ou un reste alkyle, aryle, aralkyle, alkoxy ou aralkoxy éventuellement substitué, en sorte que lors de la production de 3-chloranilines, l'un des restes $X^1$ ou $X^2$ représente le chlore et que lors de la production de 3,5-dichloranilines, $X^1$ et $X^2$ représentent tous deux du chlore,

$R^1$, $R^2$, $R^3$ sont égaux ou différents et représentent du chlore, de l'hydrogène ou un reste alkyle, aryle, aralkyle, alkoxy ou aryloxy éventuellement substitué, l'un au moins des restes $R^1$, $R^2$ ou $R^3$ représentant du chlore,

avec l'hydrogène en milieu acide en présence de métaux nobles éventuellement déposés sur des supports, sous la forme élémentaire ou sous la forme liée et, le cas échéant, en présence de solvants et/ou de diluants organiques inertes et/ou d'eau à des pressions élevées et à une température élevée, caractérisé en ce que la réaction est conduite en présence de phénols éventuellement substitués et en l'absence d'iodure d'hydrogène et/ou de composés libérant de l'iodure d'hydrogène.

2. Procédé suivant la revendication 1, caractérisé en ce qu'on utilise comme phénols éventuellement substitués, des phénols qui sont substitués une ou plusieurs fois par des groupes alkyle ayant jusqu'à 4 atomes de carbone et/ou par un halogène.

3. Procédé suivant les revendications 1 et 2, caractérisé en ce qu'on utilise le phénol, le o-, le m- et le p-crésol, le 2,3-, le 2,4-, le 2,5-, le 2,6- et/ou le 3,5-xylénol.

4. Procédé suivant les revendications 1 et 2, caractérisé en ce qu'on utilise le phénol, le o-crésol, le m-crésol, le p-crésol ou un mélange de m- et de p-crésol.

5. Procédé suivant les revendications 1 à 4, caractérisé en ce qu'on utilise les phénols éventuellement substitués en quantités de 1 à 90 % en poids par rapport au poids total du mélange réactionnel.

6. Procédé suivant les revendications 1 à 4, caractérisé en ce qu'on utilise les phénols éventuellement substitués en quantités de 30 à 70 % en poids par rapport au poids total du mélange réactionnel.

7. Procédé suivant les revendications 1 à 6, caractérisé en ce que le catalyseur à base de métal noble éventuellement déposé sur un support est séparé après la fin de la réaction et après dilution du mélange réactionnel et est réutilisé directement ou après un lavage à l'eau ou à l'acide chlorhydrique dans une réaction ultérieure.